# EUROPEAN PATENT APPLICATION

(11) **EP 2 127 686 A2**
(43) Date of publication of application: **02.12.2009**
(21) Application number: 09251113.8
(22) Date of filing: 17.04.2009
(51) Int. Cl.: A61L 2/20, A61L 2/22, A61L 2/24, A61L 9/015, A61L 9/14, F24F 3/16, A61L 101/10, A61L 101/22

(54) **Area decontamination via low-level concentration of germicidal agent**

(30) Priority: 18.04.2008 US 105919
(71) Applicant: Ethicon, Inc, Somerville, NJ 08876 (US)
(72) Inventor: Platt Jr., Robert C., Laguna Niguel, CA 92677 (US); Lin, Szu-Min, Irvine, CA 92620 (US); Lukasik, Robert G., Lake Elsinore, CA 92530 (US)
(74) Representative: James, Anthony Christopher W.P.

(57) **Abstract**

Provided is a method of decontaminating an area, and related device. The method comprises releasing a germicidal agent into the area from a germicidal source, controlling the germicidal agent concentration in the area to a predetermined concentration that does not exceed a Permissible Exposure Limit, and contacting microorganisms in the area with the germicidal agent to decontaminate the area. The device comprises a releasing mechanism configured to release a germicidal agent from a germicidal source at a predetermined concentration into the area such that the predetermined concentration of germicidal agent in the area does not exceed a Permissible Exposure Limit.

## Description

### FIELD

The present disclosure is directed to area decontamination via low-level concentration of germicidal agent, and devices for providing the same.

### BACKGROUND

The ability of microorganisms to contaminate environmental surfaces in areas such as a hospital patient room is well known. Accordingly, operators of facilities concerned with health and wellness have stringent regimens to reduce or eliminate environmental contamination to prevent transfer of microorganisms from these surfaces to customers, patients, or personnel. The primary agent by which microorganisms are transferred from environmental surfaces to patients or workers is the hands of the patients or workers. Thus, standard environmental cleaning is most concerned with high-touch surfaces, e.g., bed rails and less concerned with low-touch surfaces, e.g., floors and ceilings.

Standard procedures to address area contamination include measures, such as dusting, vacuuming, mopping and wiping low-touch areas, with or without disinfecting agents. High-touch surfaces are typically disinfected using chemical agents that are wiped or sprayed on, allowed the proper contact time to ensure germicidal activity, and then wiped off. As these high-touch surfaces are manifold in both number and complexity, the opportunity for failure to treat all surfaces properly is high. Because of the noxious character of the disinfecting agents, these regimens typically occur, for example, between patient occupancy of hospital rooms and between surgical procedures in operating rooms. Even though healthcare facilities take great care to address decontamination issues, it is not uncommon to find residual contamination after cleaning. An alternative to these treatments is area decontamination by disinfecting vapor or mist in which the area is evacuated and closed or sealed and then subjected to the disinfecting vapor or mist for a period of time. These procedures require the areas to be taken out of service for a period of time for treatment but ensure disinfection of all exposed surfaces in the treated area.

Inadequate or incomplete cleaning and disinfection allows for the possibility of transfer of infectious microorganisms from environmental surfaces to patients or workers, while facility downtime to fully address contamination issues is costly and inefficient. Accordingly, the need exists for decontamination measures beyond the current standard procedures to address biological contamination in various facilities.

### BRIEF DESCRIPTION OF THE DRAWINGS

The characteristics and advantages of the present disclosure may be better understood by reference to the accompanying drawing in which:
FIG. 1 is a schematic of a device for decontaminating an area of the present disclosure in the form of a humidifier;
FIG. 2 is a schematic of a device for decontaminating an area of the present disclosure in the form of a ozone generation device;
FIG. 3 is a schematic of a device for decontaminating an area of the present disclosure in the form of a disposable single use or reusable system;
FIG. 4 is a schematic of a device for decontaminating an area of the present disclosure in the form of a single room unit connected to a conventional HVAC system;
FIG. 5 is a schematic of a device for decontaminating an area of the present disclosure in the form of multiple room units connected to a conventional HVAC system; and
FIG. 6 is a schematic of a device for decontaminating an area of the present disclosure in the form of multiple room units connected to a conventional HVAC system.

### DETAILED DESCRIPTION

Embodiments of the present disclosure provide a device and related method of decontaminating microorganisms in an area at low concentrations of germicidal agent that does not require the area to be taken out of service. Accordingly, areas, such as hospital rooms, can be occupied continuously or nearly continuously while decontamination is underway.

Other than in the operating examples, or unless otherwise expressly specified, all of the numerical ranges, amounts, values and percentages such as those for amounts of materials, times and temperatures of reaction, ratios of amounts, and others in the following portion of the specification may be read as if prefaced by the word "about" even though the term "about" may not expressly appear with the value, amount or range. Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements. Furthermore, when numerical ranges of varying scope are set forth herein, it is contemplated that any combination of these values inclusive of the recited values may be used. The terms "one," "a," or "an" as used herein are intended to include "at least one" or "one or more," unless otherwise indicated.

Any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Embodiments of the present disclosure provide devices and method of decontaminating an area. The method comprises releasing a germicidal agent into the area from a germicidal source, controlling the germicidal agent concentration in the area to a predetermined concentration that does not exceed a Permissible Exposure Limit, and contacting microorganisms in the area with the germicidal agent to decontaminate the area.

The area may be the air and/or surfaces of any room or facility known to those of ordinary skill in the art that are subject to growth or development of microorganisms, and where it is desired and/or required to maintain sanitary conditions. For example, areas contemplated by the present disclosure include an office, a home, a hospital facility, a hospital patient room, an intensive care unit, an ambulance, an operating room, an airport, a health club facility, a restaurant, a restroom facility, a laboratory, an animal facility, such an animal kennel, an animal hospital, or an animal shelter, and combinations of any thereof. The area may be enclosed or semi-enclosed, such as in situations were a door or window is ajar or partially ajar.

The germicidal agent may be released into the area from a germicidal source in the form of a gas, a vapor, a mist, or combinations of any thereof. As used herein, the term "gas" is defined as a state of matter in which molecules do not condense into liquid or solid under normal conditions, i.e., standard temperature and pressure. The term "vapor" is defined as the air dispersion of molecules of a substance that is liquid or solid in its normal state, *i*.*e*., at standard temperature and pressure. As used herein, the term "mist" refers to small droplets of liquid. As employed herein, the term "mist" is used as a generic expression to include all forms of droplets suspended in a gas phase composition, including aerosols, fogs, and the like.

The germicidal agent may be released into the area at concentrations whereby humans or animals can be chronically and safely exposed, and which are suitable to reduce, eliminate or weaken microorganisms in the air and/or on surfaces of the area. Microorganisms contemplated by the present disclosure for decontamination include various infectious agents, such as viruses, vegetative bacteria, fungi, mycobacteria, bacterial spores, and combinations thereof, and in particular, include, for example, Methicillin-resistant *Staphylococcus aureus* (MRSA), Vancomycin-resistant *enterococcus* (VRE), Norovirus, *S. aureus, C. difficile*, and the like.

The germicidal agent may be, for example, hydrogen peroxide or ozone. When hydrogen peroxide is employed, the germicidal source may be, for example, a liquid hydrogen peroxide solution or a solid peroxide complex. The liquid hydrogen peroxide solution may be, for example, H₂O/H₂O₂ solutions. In certain embodiments, the hydrogen peroxide solutions may contain 30% water, in certain embodiments 60% water, in certain other embodiments no more than 90% water, by weight. In certain embodiments, hydrogen peroxide solutions may be employed to generate a substantially non-aqueous (*i*.*e*., substantially anhydrous) source of hydrogen peroxide gas, vapor, and/or mist which is processed during vaporization to remove water, such as under vacuum. Thus, in certain embodiments where an aqueous hydrogen peroxide complex is used, the aqueous complex may be converted to a substantially non-aqueous hydrogen peroxide germicidal agent while carrying out the process, as described in U.S. Patent Nos. 5,667,753, 5,674,450, and 5,785,934, which are incorporated by reference herein in their entirety. As discussed herein, the germicidal source may be heated to facilitate the release of germicidal agent.

The solid peroxide complex has the advantage of releasing substantially no water into the atmosphere with the peroxide. A substantially non-aqueous (i.e., substantially anhydrous) source of hydrogen peroxide generally releases a substantially non-aqueous hydrogen peroxide gas or vapor. In one embodiment, the substantially non-aqueous hydrogen peroxide vapor is produced directly from a substantially nonaqueous hydrogen peroxide complex. Various solid peroxide complexes are contemplated by the present disclosure. In one embodiment, for example, the germicidal agent is hydrogen peroxide released from a urea hydrogen peroxide complex. Urea hydrogen peroxide complex is available in tablet form from Fluka Chemical Corp., Ronkonkoma, N.Y. and in powder form from Aldrich Chemical Co., Milwaukee, Wis. This complex is also known as urea peroxide, hydrogen peroxide urea complex, peroxide urea, peroxide urea adduct, urea peroxide adduct, percarbamide, carbamide perhydrate, and carbamide peroxide. As used herein, the term "urea peroxide" encompasses all of the foregoing terms. Various other hydrogen peroxide complexes may be employed in the present disclosure. For example, suitable hydrogen peroxide complexes contemplated by the present disclosure include a poly(vinyl alcohol)-hydrogen peroxide complex, a poly(vinyl methyl ether)-hydrogen peroxide complex, a poly(vinyl methyl ketone)-hydrogen peroxide complex, a poly(acrylic acid)-hydrogen peroxide complex, a poly(vinyl acetate) hydrogen peroxide complex, a cellulose acetate hydrogen peroxide complex, a sodium alginate hydrogen peroxide complex, a cellulose sulfate, sodium salt, hydrogen peroxide complex, a glycine-hydrogen peroxide complex, poly(4-vinylpyridine) hydrogen peroxide complex, a histamine hydrogen peroxide complex, a propionamide hydrogen peroxide complex, a 1,3-dimethylurea hydrogen peroxide complex, a biuret hydrogen peroxide complex, a polyacrylamide hydrogen peroxide complex, a nylon 6 hydrogen peroxide complex, a nylon 6,6 film hydrogen peroxide complex, a polyetherpolyurethane hydrogen peroxide complex, and a rubidium carbonate hydrogen peroxide complex. When solid peroxide complexes are employed, some water may be present in the system. Some water may derive from the decomposition of hydrogen peroxide to form water and oxygen as byproducts and some hydrogen binding of this water to the complex can occur.

Other hydrogen peroxide solutions or complexes suitable for use in the present disclosure are those complexes described in U.S. Patent Nos. 5,667,753, 5,674,450, and 5,785,934.

When the germicidal agent is ozone, the germicidal source is oxygen. In certain embodiments, the oxygen may be supplied by air, a source of pure oxygen, or a combination thereof.

When released into the area, the germicidal agent may be controlled within any suitable concentration at which humans or animals can be chronically exposed. Typically, the concentration of the germicidal agent is controlled in the area to a predetermined level that does not exceed a "low- level" concentration, or "Permissible Exposure Limit." As used herein, "Permissible Exposure Limit" (PEL) refers to regulatory limits on the amount or concentration of a substance in the air, as imposed by a government or national health authority. For example, the Occupational Safety and Health Administration ("OSHA") sets PELs to protect workers against the health effects of exposure to hazardous substances. For hydrogen peroxide, the PEL set by OSHA is 1.0 ppm. In like manner the PEL of ozone, as set by OSHA, is 0.1 ppm. Regulatory bodies in other countries may have different PEL's.

Accordingly, in certain other embodiments of the subject application, the germicidal agent may be controllably released in the area at various predetermined concentrations that are no greater than imposed PEL limits. In other embodiments, the predetermined concentration is no greater than one-half of the Permissible Exposure Limit. In still other embodiments, the predetermined concentration is no greater than one-third of the Permissible Exposure Limit. For example, in certain embodiments, and for the OSHA PEL for hydrogen peroxide of 1.0 ppm, the germicidal agent may be controllably released at 1.0 ppm or less, in other embodiments at 0.75 ppm or less, in still other embodiments at 0.5 ppm or less, and in other embodiments at 0.3 ppm or less. In embodiments where the germicidal agent is ozone for which the OSHA PEL is 0.1 ppm, the ozone is controllably released at 0.1 ppm or less, and in some embodiments at 0.05 ppm or less. In certain embodiments, these concentration levels are controlled and/or maintained during standard operation of the device (i.e. between the times conventionally known as "startup" and "shutdown").

The germicidal agent may be released into the area at various frequencies that allow concentration control at chronic levels. For example, it is contemplated that release of the germicidal agent into the area may be continuous, intermittent, periodic, or combinations thereof. Continual and controlled release of the germicidal agent allows manual and substantially unsupervised operation of the decontamination device. In other embodiments, release of the germicidal agent may be intermittent or periodic. It is contemplated that intermittent or periodic release of the germicidal agent may be made at various stages of decontamination, such as after a low-level concentration of the germicidal agent has reached a predetermined set point after continual operation. As described in further detail herein, control of the low levels of the germicidal agent in the area to a predetermined concentration may be performed by various methods, such as by positioning optional sensors and monitors in the area. In this manner, the release may be interrupted for a period of time based on feedback controls through sensor devices and/or programmable monitoring that allows the release of germicidal agent to be temporarily suspended, such as by turning off a power supply, such as a heater, and/or by shutting down a distribution mechanism, such as a fan or blower, in the device. The intermittent or periodic release may be based on various set points, discussed herein, which may be incorporated into the device. In addition, duty cycle may also be varied. For example, rather than continuous exposure, release may be restricted to a certain number of hours per day, a number of days per week, or by time of day. For instance, release may occur for 8 hours per day or, for example, in a restroom, only late at night, to minimize human exposure.

Various set points may be incorporated into the device that provide selections of, for example, running time (on time, off time, or on/off times), rate (amount released per unit time), germicidal agent concentration, and/or area size or condition (seal/closed and evacuated area), and combinations thereof, such that the germicidal agent may be controllably release within the predetermined low concentration levels. In this manner, an operator can select from one or more operation settings on the device that control the release of germicidal agent into the area. The various set points allow the device to be more conveniently operated in areas that may demand adjustment to the operating conditions.

In certain embodiments, it is contemplated that the methods of decontamination may be employed that control concentration of the germicidal agent above and below the PEL. For example, in one embodiment, a method of decontaminating an area comprises controlling a concentration of the germicidal agent in the area to a predetermined level that does not exceed the PEL while the area is in standard operation, and then increasing the concentration of the germicidal agent in the area to a level that exceeds the PEL for a period of time for acute decontamination. Accordingly, the device may be configured to release a germicidal agent at a predetermined concentration into the area such that in standard operation does not exceed a PEL, but for acute decontamination may exceed the PEL for short periods of time. The used herein, the term "standard operation" is defined as those times where the area is occupied by humans and/or animals and is being used for its intended and designated purpose. Standard operation may be, for example, during normal business hours. The term "acute decontamination" is defined as decontamination with germicidal agent at concentrations that greatly exceed PEL for short periods of time. For example, when hydrogen peroxide is employed as the germicidal agent, acute decontamination is typically conducted at levels ranging from 30 to 1500 ppm for up to 6-8 hours. During times when the area is not in standard operation, the area may be decontaminated with germicidal agent at concentrations above the PEL. In this manner, decontaminating an area may be made more efficient during periods outside normal business hours or when the area is not in use, such as between procedures, at night, during weekends, over holiday closings, and the like.

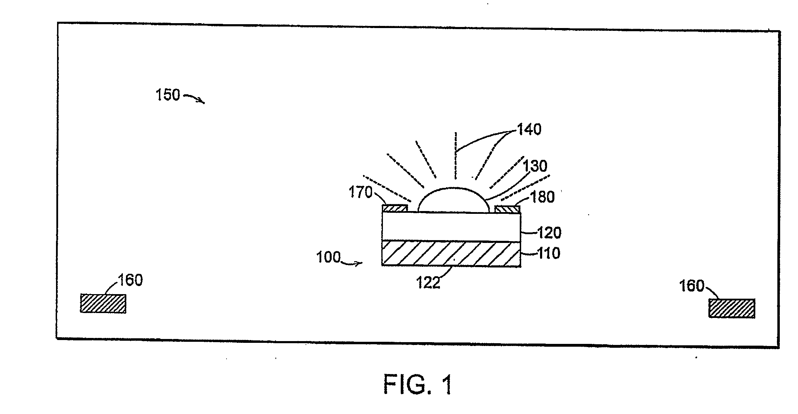

Turning now to the drawings, FIG. 1 is a schematic of a device 100, in the form of a humidifier, for decontaminating an area 150. The device 100 comprises a germicidal source 110 positioned within a housing 120, and a releasing mechanism 130 in communication with the housing 120. The releasing mechanism 130 is configured to release the germicidal agent 140 from the germicidal source 110 at a predetermined concentration into the area 150 such that the predetermined concentration of germicidal agent 140 in the area 150 does not exceed the PEL.

The housing 120 may comprise a support 122 for holding the germicidal source 110 when the germicidal source 110 is hydrogen peroxide, and may be, for example, a tank, a plate, or a pan. As illustrated, the housing 120 may comprise, for example, a metal support, such as a pan, *e.g.*, an aluminum pan. The germicidal agent 140 may be produced from the germicidal source 110 in the form of a gas, a vapor, and/or a mist by various mechanisms, such as a gas generator, a mist generator, a humidifier (illustrated), a heater, and combinations of any thereof. Generally, as used herein, the "releasing mechanism" may be any device or mechanism that allows or assists the germicidal agent in traversing from the germicidal source into the area. For example, the releasing mechanism 130 may comprise a gas permeable screen or cloth that allows the germicidal agent to diffuse into the area 150. In certain embodiments, the releasing mechanism 130 may incorporate a fan or blower to assist in diffusing the germicidal agent 140 throughout the area 150. In small areas, or when the device 100 is incorporated into an existing air circulation system, such as an HVAC system, discussed below, a fan or blower may not be necessary. The releasing mechanism 130 may also comprise a flow controller, such as a controller commercially available from Bronkhorst High-Tech B.V., Netherlands. As used herein, a flow controller is a device or material that by its composition, geometry, or mechanical action controls the release of germicidal agent 140 into the area 150.

Control of the low levels of the germicidal agent 140 in the area 150 to a predetermined concentration may be performed by various methods, such as by positioning one or more optional sensors 160 in the area 150. The sensor 160 may be positioned on the housing 120 or may be remotely located in the area 150 to provide, for example, programmable monitoring of the concentration levels of the germicidal agent 140 in the area 150 and provide a feedback control mechanism to, for example, a flow controller configured to control the release and concentration of the germicidal agent in the area 150. Release of germicidal agent 140 may be continuous, intermittent, and/or periodic as set forth herein. The release frequency of the germicidal agent 140 may be based on various set points that are selected from a set point controller 170. The set points may be programmable or manual and may include, for example, running time, germicidal agent concentration, area size, and combinations of any thereof. The number of sensors 160 employed and their orientation may vary depending upon the desired uniformity of germicidal agent concentration throughout the area 150. Depending upon such factors as the configuration and positioning of the device 100 within the area 150, it is contemplated that the releasing mechanism 130 can be controlled by a microprocessor and configured so that, for example, multiple flow controllers may be independently controlled such that one or more flow controllers directed to certain locations of the area 150, or in certain rooms of a facility, may operate to emit different amounts of germicidal agent 140 in order to create greater uniformity of germicidal agent 140, or, alternatively, to provide higher or lower concentrations of germicidal agent 140 in particular selected locations, based on sensor feedback controls.

As illustrated, the device 100 may comprise other optional features, such as a use-life indicator 180, to indicate, for example, when the amount of germicidal source 110 in the housing 120 has been expended. The use-life indicator could be a liquid level sensor, a hydrogen peroxide detector of the remote sensor.

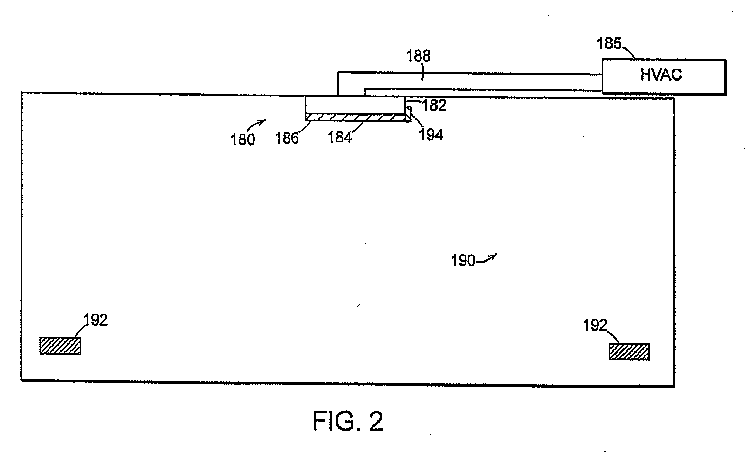

FIG. 2 is a schematic of a device 180, in the form of an ozone generation device connected to a conventional HVAC system 185 for decontaminating an area 190. The device 180 comprises a housing 182, and a releasing mechanism 184 in communication with the housing 182. The releasing mechanism 184 is configured to release the germicidal agent at a predetermined concentration into the area 190 such that the predetermined concentration of germicidal agent in the area 190 does not exceed the PEL. When the germicidal agent is ozone, the releasing mechanism 184 may comprise an ozone generation device 186, such, for example, an arc discharge, a corona discharge, or an ultraviolet-emitting (UV) source, such as a UV lamp. For example, ozone gas within the predetermined and controlled concentrations set forth herein may be produced using a corona discharge generator. In one embodiment, the corona discharge generator may comprise a sealed glass tube that was filled with a gas. An electrical conductor rod passed through the glass tube at one end may extend the entire length of the tube. A perforated, metal grid may surround the tube so that when a high voltage is applied across the rod and the grid, a corona is developed between the glass tube and the grid. Oxygen passing through the corona is ionized to produce ozone. Ozone produced by the corona may be dispersed into the area by virtue of the ion wind produced by the corona, and/or by an optional fan or blower. Concentration of the ozone may be controlled through the use of sensors and programmable monitors, as discussed herein, to control a predetermined concentration no greater than the PEL ranges set forth herein. In certain embodiments of the present disclosure, relative humidity levels are maintained above 20%, and in some embodiments are maintained at levels ranging from 70% to 90%. Humidity can be controlled by a humidifier and humidity sensor.

In certain embodiments, forced and/or heated air may be supplied to the device 180 by the existing HVAC system 185 via an existing ventilation system 188. Accordingly, in certain embodiments, separate heating and distribution systems, if desired, may be provided by the existing HVAC system 185. The device 180 may be configured for releasing germicidal agent, such as ozone, into the area 190 in the form of ozone gas.

As set forth herein, the concentration of the ozone gas may be controlled at low levels in the area 190 to a predetermined concentration through the use of options sensors 192 that provide a feedback control mechanism to control the release of ozone gas in the area 190. Release of ozone gas may be continuous, intermittent, and/or periodic by a set point controller 194, in a manner discussed herein.

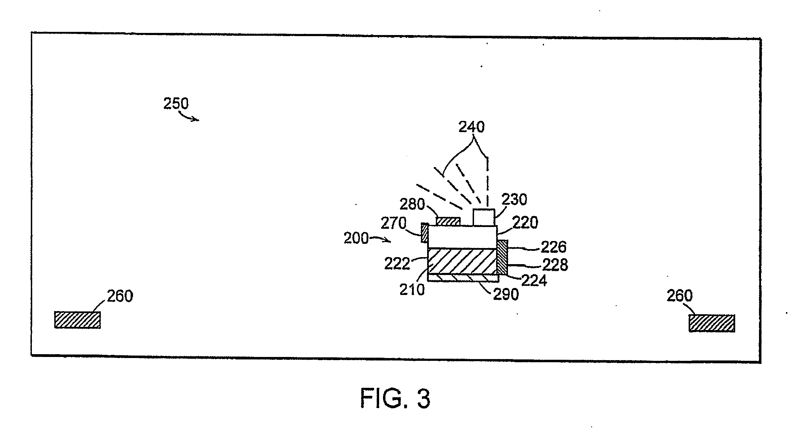

FIG. 3 is a schematic of a device 200, in the form of a wall-mount disposable single use or reusable system, for decontaminating an area 250. The device 200 comprises a germicidal source 210 positioned within a housing 220, and a releasing mechanism 230 in communication with the housing 220. The releasing mechanism 230 is configured to release the germicidal agent 240 from the germicidal source 210 at a predetermined concentration into the area 250 such that the predetermined concentration of germicidal agent 240 in the area 250 does not exceed the PEL.

The housing 220 may comprise a support 222 for holding the germicidal source 210 when the germicidal source 210 is solid peroxide complex or liquid hydrogen peroxide solution, and may be, for example, a tank, a plate, or a pan. As illustrated, the housing 220 may comprise, for example, a metal support, such as a pan, *e.g.*, an aluminum pan, and an optional heater 290 for heating the germicidal source 210 to release the germicidal agent in the form of a gas, a vapor, and/or a mist. When the optional heater 290 is employed, a temperature monitor, such as a thermometer, may be in communication with the housing 220 to monitor and control the support temperature. The germicidal source 210 may be placed directly on or in the support 222. Alternatively and optionally, if the germicidal source is a solid peroxide complex, in order to provide even heating of the solid peroxide complex, the peroxide complex can be placed between one or more aluminum screens (not shown) placed on top of a support 222. The peroxide complex may be covered with a gas permeable layer such as a layer of medical grade TYVEK, commercially available from DuPont de Nemours & Co., Wilmington, DE, or SPUNGUARD, commercially available from Kimberly-Clark Co., Dallas, TX, so that the germicidal agent 240 released from the germicidal source 210 may pass through the covering before diffusing into the area 250 by a releasing mechanism 230.

As set forth herein, the concentration of the germicidal agent 240 may be controlled at low levels in the area 250 to a predetermined concentration through the use of options sensors 260 provide a feedback control mechanism to control the release of germicidal agent 240, as discussed herein. Release of germicidal agent 240 may be continuous, intermittent, and/or periodic by a set point controller 270, in a manner discussed herein. As illustrated, the device 200 may have other optional features, such as a use-life indicator 280.

As illustrated, the device 200 may include an insulating housing (not shown), from which may extend terminal portions 226, 228 of a standard wall-mounted plug 224 which can be plugged into mating sockets of a wall outlet, such as a standard 120 volt wall outlet. The act of plugging the device 200 into the mating socket of the power supply activates the power supply to heat the germicidal source 210 and to release the germicidal agent 240. In certain embodiments, the voltage of the device is only applied when the device 200 is plugged into the power supply. If desired, the device 200 can be locked in position with suitable locating screws or fittings. In other embodiments of the present disclosure, the device may be battery operated. It is contemplated that the device 200 may be reusable such that when the germicidal source 210 is expended, the support 222, such as in the form of a replacement cartridge, may be removed and replaced. In the alternative, the device 200 may be a single use device that is disposed of when the germicidal source 210 is spent.

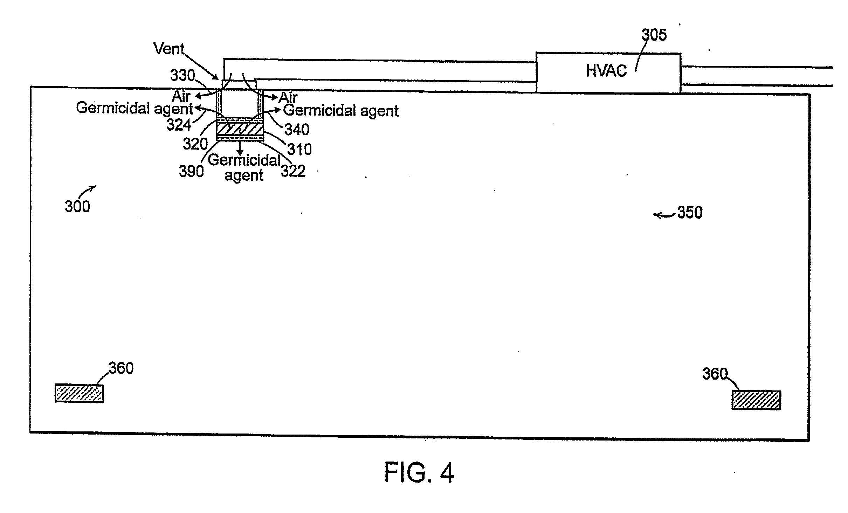

FIG. 4 is a schematic of a device 300, in the form of a room unit connected to a conventional HVAC system 305, for decontaminating an area 350, such as a single room. The device 300 comprises a germicidal source 310 positioned within a housing 320, and a releasing mechanism 330 in communication with the housing 320. The releasing mechanism 330 is configured to release the germicidal agent 340 from the germicidal source 310 at a predetermined concentration into the area 350 such that the predetermined concentration of germicidal agent 340 in the area 350 does not exceed the PEL.

The housing 320 may comprise a support 322 for holding the germicidal source 310, which may be a solid peroxide complex or liquid hydrogen peroxide solution, an optional heater 390, as discussed herein, for heating the germicidal source, or an ozone generating device, such as those devices set forth herein. The support 322 may include a gas permeable enclosure to package the germicidal source 310. In certain embodiments, forced and/or heated air may be supplied to the device 300 by the existing HVAC system 305 via an existing ventilation system. Accordingly, in certain embodiments, a separate heating and distribution systems, if desired, may be provided by the existing HVAC system 305. As illustrated, the housing 320 may comprise multiple controllable openings 324 to direct the air flow and for releasing germicidal agent into the area 350 in the form of a gas, a vapor, and/or a mist. Accordingly, the releasing mechanism 330 may comprise, for example, the openings 324 and the forced air of the HVAC system 305.

As set forth herein, the concentration of the germicidal agent 340 may be controlled at low levels in the area 350 to a predetermined concentration through the use of options sensors 360 that provide a feedback control mechanism to control the release of germicidal agent 340, such as through flow controllers, configured to control the release of germicidal agent through the openings 324 and the concentration of the germicidal agent 340 in the area 350. Release of germicidal agent 340 may be continuous, intermittent, and/or periodic by a set point controller (not shown), in a manner discussed herein.

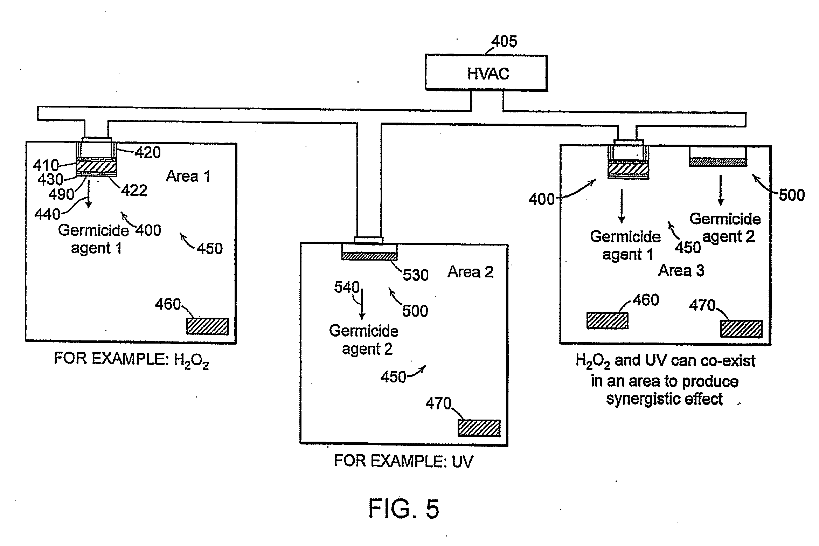

FIG. 5 is a schematic of one or more devices 400, 500, in the form of a multiple room units connected to a conventional HVAC system 405, for decontaminating one or more areas 450. The device 400 may comprise a germicidal source 410 positioned within a housing 420, and a releasing mechanism 430 in communication with the housing 420. The releasing mechanism 430 is configured to release the germicidal agent 440 from the germicidal source 410 at a predetermined concentration into the area 450 such that the predetermined concentration of germicidal agent 440 in the area 450 does not exceed the PEL.

The housing 420 may comprise a support 422 for holding the germicidal source 410, which may be a solid peroxide complex or liquid hydrogen peroxide solution, an optional heater 490, as discussed herein, for heating the germicidal source, or an ozone generating device, such as those devices set forth herein. As illustrated, the releasing mechanism 430 may release germicidal agent 440 into the area 450 in the form of a gas, a vapor, and/or a mist. Heat and forced air, if desired, can be supplied by the existing HVAC system 405, as discussed above.

As illustrated, one or more regions of area 450 may also, or alternatively, comprise an ozone generation device 500 connected to the HVAC system 405, for decontaminating a second region of the area 450. The device 500 comprises a releasing mechanism 530 configured to release the germicidal agent 540, such as ozone gas, at a predetermined concentration into the area 450 such that the predetermined concentration of germicidal agent 540 in the area 450 does not exceed the PEL, in a manner set forth here. In certain embodiments, forced and/or heated air may be supplied to the device 500 by the existing HVAC system 405 via an existing ventilation system. Accordingly, in certain embodiments, separate heating and distribution systems, if desired, may be provided by the existing HVAC system 405.

In certain embodiments, and as illustrated, the area 450 may include one or more regions, with at least one region comprising one or more devices 400, 500 such that device 400 and device 500 work independently or cooperatively to control the level of germicidal agent in the area 450. For example, one region may include only device 400, such as a humidifier, that controls the level of germicidal agent, such as hydrogen peroxide, as provided herein. A second region may include only device 500, that may be the same or different than device 400, such as an ozone generator, that controls the level of ozone gas, as provided herein. A third region may include more than one device 400, 500, that may be the same or different, such as, for example, a humidifier and an ozone generator. In the third region, the humidifier controls the level of germicidal agent 440, and the ozone generator controls the level of germicidal agent 540 in the area. In this manner, it is contemplated that multiple same or different devices 400, 500 may co-exist in a region of the area that may produce a synergistic effect therein.

The concentration of the germicidal agent 440, 540 may be controlled at low levels in the area 450 to a predetermined concentration through the use of optional sensors 460, 470 that provide a feedback control mechanism to control the release of germicidal agent 440, 540, respectively. For example, sensor 460 may be a hydrogen peroxide sensor, and sensor 470 may be an ozone sensor. As discussed herein, the number of sensors 460, 470 employed and their orientation may vary depending upon the desired uniformity of germicidal agent concentration throughout the area 450. Depending upon such factors as the configuration and positioning of the device 400 within the area 450, it is contemplated that the releasing mechanism 430, 530 can be controlled by a microprocessor and configured so that, for example, a flow restrictor in each room of the area 450 may be independently controlled such that germicidal agent 440, 540 may be provided at higher or lower concentrations in particular selected locations, based on sensor feedback controls. Furthermore, as discussed herein, release of germicidal agent 440, 540 may be continuous, intermittent, and/or periodic as set forth here by a set point controller (not shown), in a manner discussed herein.

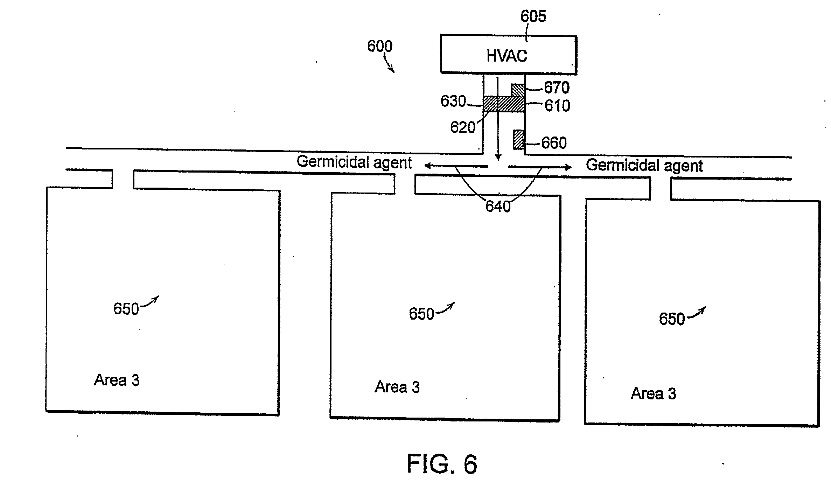

FIG. 6 is a schematic of a device 600 for decontaminating an area 650 of the present disclosure in the form of multiple room units connected to a conventional HVAC system. The device 600 may comprise a germicidal source 610 positioned within a housing 620, and a releasing mechanism 630 in communication with the housing 620. The releasing mechanism 630 may be configured to release the germicidal agent 640 from the germicidal source 610 at a predetermined concentration into the area 650 such that the predetermined concentration of germicidal agent 640 in the area 650 does not exceed the PEL.

The housing 620 may contain the germicidal source 610, which as discussed herein, may be, for example, a solid peroxide complex, liquid hydrogen peroxide solution, or an ozone generating device. As illustrated, the releasing mechanism 630 may release germicidal agent 640 into the area 650 in the form of a gas, a vapor, and/or a mist. Heat and forced air, if desired, can be supplied by the existing HVAC system 605, as discussed above. As illustrated, the device 600 need not be positioned within the area 650, but, instead, may be positioned in-line with an existing ventilation system.

The concentration of the germicidal agent 640 may be controlled within the PEL ranges set forth herein in the area 650 to a predetermined concentration through the use of optional sensors 660 that provide a feedback control mechanism to control the release of germicidal agent 640. As discussed herein, the number of sensors 660 employed and their orientation may vary depending upon the desired uniformity of germicidal agent concentration throughout the area 660. As illustrated, the sensor 660 may be positioned in-line with an existing ventilation system, near the germicidal source, so that germicidal agent concentration is uniformly distributed to the various regions of the area 650. Depending upon such factors as the configuration and positioning of the device 600, it is contemplated that the releasing mechanism 630 can be controlled by a microprocessor and configured so that, for example, a flow restrictor may be independently controlled to release germicidal agent 640 at higher or lower concentrations into the area 650, based on sensor feedback controls. Furthermore, as discussed herein, release of germicidal agent 640 may be continuous, intermittent, and/or periodic as set forth here by a set point controller 670, in a manner discussed herein. The embodiments shown in Figures 5 and 6 may be combined to ensure the desired germicide concentrations are achieved in all areas.

As set forth in the examples, below, by providing a continual chronic flow of low concentration germicidal agent to the area, microorganism contamination in areas prone to their development may be reduced or substantially eliminated without reducing or eliminating the need for facility downtime. Embodiments of the present disclosure could also provide increased flexibility and efficiency relative to present decontamination systems by providing the option to greatly increase the concentration of the germicidal agent from the chronic low concentrations set forth herein to a concentration above the PEL to provide acute treatment of an area for a period of time. This would require the area to be taken out of service for the duration of the acute treatment. Thereafter, the system could, again, be run at low chronic concentration levels.

Accordingly, embodiments of the present disclosure provide a device and related method of decontamination of microorganisms in an area at low concentrations of germicidal agent that does not require the area to be taken out of service. Accordingly, areas, such as hospital rooms, can be occupied continuously or nearly continuously while decontamination is underway.

The invention will be further described by reference to the following examples. The following examples are merely illustrative of the invention and are not intended to be limiting. Unless otherwise indicated, all parts are by weight.

### EXAMPLES

### Example 1

An experiment was conducted employing hydrogen peroxide vapor released from a urea complex at a nominal level of 0.6 ppm to demonstrate a reduction of viable G. *stearothermophilus* spores on a stainless steel surface as follows. Stainless steel scalpel blades were inoculated with 10 µL of inoculum containing 6 x 10⁴ spores. The inoculum was then dried on the blade surface. Nine inoculated blades where placed in a 1 ft³ sealed chamber constructed of acrylic. In the chamber a urea complex was confined in a glass tube with a flow restriction that allowed the concentration of peroxide in the chamber to be controlled. Three identically-inoculated blades where placed inside a covered petrie dish and placed near the chamber to serve as controls. At three day intervals, three blades were retrieved from the chamber as well as one control. These blades were subjected to a standard recovery, plating and incubation procedure from which the viable spore count and log reduction could be determined. Log reductions as well as the average high and low peroxide concentrations during exposure are shown in Table 1.

**Table 1**

| | AVERAGE LOG REDUCTION | [H₂O₂] Average, low, high |
|---|---|---|
| | | during exposure period |
| After 3 Days Exposure | 1.5 | 0.56, 0.40, 0.83 ppm |
| After 6 Days Exposure | 2.7 | 0.66, 0.40, 0.83 ppm |
| After 9 Days Exposure | ≥4.8 | 0.66, 0.40, 0.95 ppm |

The results for Table 1 demonstrate an approximate 0.5 log reduction in *G. stearothermophilus* spores per day with a nominal concentration of 0.6 ppm of hydrogen peroxide. *G. stearothermophilus* spores have the highest known resistance to hydrogen peroxide. The effectiveness of the method against non-spore forming microorganisms such as *S. aureus* and even spore former *C. difficile* is expected to be greater.

### Example 2

To demonstrate the dependence of log reduction of *G. stearothermophilus* spores on peroxide concentration, scalpel blades, prepared as described in Example 1, were exposed to a nominal H₂O₂ concentration of 0.35 ppm. In this case, the inoculated blades were exposed serially which resulted in some variation in the average peroxide concentration during the exposure period. Log reductions after 3, 6 and 9 days of exposure as well as the average, high and low peroxide concentrations during exposure are shown in Table 2.

**Table 2**

| | AVERAGE LOG REDUCTION | [H₂O₂] Average, low, high during exposure period |
|---|---|---|
| After 3 Days Exposure | 1.12 | 0.35, 0.2, 0.5 ppm |
| After 7 Days Exposure | 1.83 | 0.30, 0.2, 0.5 ppm |
| After 9 Days Exposure | ≥4.0 | 0.39, 0.2, 0.6 ppm |

The concentrations shown above are averages over the entire exposure time. As demonstrated, some variation in 0.35 ppm nominal concentration exists, but the data in the Tables indicates higher concentration produces higher kill rates of microorganisms.

It will be appreciated by those skilled in the art that changes could be made to the embodiments described above without departing from the broad inventive concept thereof. It is understood, therefore, that this invention is not limited to the particular embodiments disclosed, but it is intended to cover modifications that are within the spirit and scope of the invention, as defined by the appended claims.

## Claims

1. A method of decontaminating an area, comprising:
releasing a germicidal agent into the area from a germicidal source;
controlling the germicidal agent concentration in the area to a predetermined concentration that does not exceed a Permissible Exposure Limit; and
contacting microorganisms in the area with the germicidal agent to decontaminate the area.

2. The method of claim 1, wherein the releasing occurs in a form selected from the group consisting of a gas, a vapor, a mist, and combinations of any thereof.

3. The method of claim 1, wherein the germicidal agent is selected from the group consisting of hydrogen peroxide and ozone.

4. The method of claim 1, wherein the germicidal agent is hydrogen peroxide and the germicidal source is selected from the group consisting of a hydrogen peroxide solution and a solid peroxide complex.

5. The method of claim 1, wherein the germicidal agent is ozone and the germicidal source is oxygen.

6. The method of claim 1, wherein the controlling occurs at a frequency selected from the group consisting of continuous, intermittent, periodic, and combinations of any thereof.

7. The method of claim 6, further comprising adjusting the frequency by at least one of a programmable and a manual selectable set point.

8. The method of claim 1, wherein the area is selected from the group consisting of a room, an office, a home, an airport, a hospital facility, a hospital patient room, an intensive care unit, an ambulance, an operating room, a health club facility, a restaurant, a laboratory, a restroom facility, an animal facility, and combinations of any thereof.

9. The method of claim 1, wherein the microorganism is selected from the group consisting of a virus, a vegetative bacteria, a fungi, a mycobacteria, a bacterial spore, and combinations of any thereof.

10. A method of decontaminating an area, comprising:
releasing a germicidal agent into an area from a germicidal source;
controlling a concentration of the germicidal agent in the area to a predetermined level in standard operation that does not exceed a Permissible Exposure Limit;
increasing the concentration of the germicidal agent in the area to a level that exceeds the Permissible Exposure Limit for acute decontamination for a period of time; and
contacting microorganisms in the area with the germicidal agent to decontaminate the area.

11. A device for decontaminating an area, comprising:
a releasing mechanism configured to release a germicidal agent from a germicidal source at a predetermined concentration into the area such that the predetermined concentration of germicidal agent in the area does not exceed a Permissible Exposure Limit.

12. The device of claim 11, wherein the germicidal agent is selected from the group consisting of hydrogen peroxide and ozone.

13. The device of claim 11, wherein the germicidal agent is hydrogen peroxide and the germicidal source is selected from the group consisting of a hydrogen peroxide solution and a solid peroxide complex.

14. The device of claim 11 wherein the germicidal agent is ozone and the germicidal source is oxygen.

15. The device of claim 11, wherein the germicidal agent is released into at least one form selected from the group consisting of a gas, a vapor, a mist, and combinations of any thereof.

16. The device of claim 15, wherein the germicidal agent is produced by a mechanism selected from the group consisting of a gas generator, a mist generator, a humidifier, a heater, a UV-emitting device, an arc discharge, and a corona discharge, and combinations of any thereof.

17. The device of claim 11, further comprising a controller configured to adjust the operation of the device to a frequency selected from the group consisting of continuous, intermittent, periodic, and combinations of any thereof.

18. The device of claim 17, wherein the controller is configured to adjust the frequency by at least one of a programmable and a manual selectable set point.

19. The device of claim 11, further comprising a sensor configured to provide feedback control.

20. The device of claim 19, wherein the sensor is located on the device or remotely located in the area.

21. The device of claim 11, wherein the area is selected from the group consisting of a room, an office, a home, an airport, a hospital facility, a hospital patient room, an intensive care unit, an ambulance, an operating room, a health club facility, a restaurant, a laboratory, a restroom facility, an animal facility, and combinations of any thereof.

22. The device of claim 11, wherein the microorganism is selected from the group consisting of a virus, a vegetative bacteria, a fungi, a mycobacteria, a bacterial spore, and combinations of any thereof.

23. The device of claim 11, further comprising a flow controller configured to control the concentration of the germicidal agent in the area.

24. A HVAC system comprising the device of claim 11.

25. The device of claim 11 further comprising a fan or blower.

26. The device of claim 11 further comprising a use life indicator.

27. A device for decontaminating an area, comprising:
a releasing mechanism configured to release a germicidal agent from a germicidal source at a predetermined concentration into the area such that in standard operation does not exceed a Permissible Exposure Limit, the releasing mechanism further configured to release germicidal agent from the germicidal source at a concentration that exceeds the Permissible Exposure Limit for acute decontamination for a period of time.
